# FASCICULE DE BREVET EUROPEEN

(11) **EP 4 175 761 B1**
(45) Date de publication et mention de la délivrance du brevet: **04.09.2024**
(21) Numéro de dépôt: 21748907.9
(22) Date de dépôt: 01.07.2021
(51) Int. Cl.: B05B 12/08, G01K 11/12, A61M 15/08

(54) **PROCÉDÉ ET DISPOSITIF D'ANALYSE D'UN DISPOSITIF DE PULVÉRISATION DE PRODUIT FLUIDE PHARMACEUTIQUE**
VERFAHREN SOWIE VORRICHTUNG ZUR KONTROLLE EINER SPRÜHVORRICHTUNG EINES PHARMAZEUTISCHEN FLÜSSIGEN PRODUKTS
METHOD AND DEVICE FOR MONITORING A PHARMACEUTICAL FLUID SPRAYING DEVICE

(30) Priorité: 01.07.2020 FR 2006943
(43) Date de publication de la demande: 10.05.2023
(73) Titulaire: APTAR France SAS, 27110 Le Neubourg (FR)
(72) Inventeur: LOISEL, Vincent, 76240 Bonsecours (FR)
(74) Mandataire: CAPRI
(86) Numéro de dépôt international: PCT/FR2021/051204
(87) Numéro de publication internationale: WO 2022/003294

(56) Documents cités:
- EP-A1- 3 047 912
- JP-A- H0 599 802
- JP-A- S54 127 347

## Description

La présente invention concerne un dispositif et un procédé d'analyse de spray généré par un dispositif de pulvérisation de produit fluide pharmaceutique.

Les dispositifs de pulvérisation de produit fluide pharmaceutique sont bien connus. Ils comportent généralement une tête de pulvérisation pourvue d'un orifice de pulvérisation, assemblée sur un réservoir contenant le produit fluide à distribuer. En particulier dans des applications de pulvérisation nasale, l'efficacité thérapeutique du produit fluide pulvérisé peut dépendre des propriétés du spray généré lors de l'actionnement du dispositif. De manière connue, à la fin de la chaîne de montage, c'est-à-dire lorsque le dispositif de pulvérisation est assemblé, et juste avant d'être expédié chez le fabriquant du produit fluide pharmaceutique pour y être assemblé sur un réservoir correspondant, un certain nombre d'échantillons de dispositifs assemblés sont testés en laboratoire pour vérifier si les propriétés du spray correspondent au cahier des charges prédéfini.

Un inconvénient de ce système est qu'il concerne des dispositifs assemblés, et donc destructeur de ces dispositifs qui ne pourront plus, après avoir été testés, être livrés au client.

De plus, ce système impose une vérification humaine des dispositifs testés, et n'est donc pas totalement automatisable.

Pour surmonter cet inconvénient, le document WO2018130791 propose la visualisation par strioscopie d'un flux d'air comprimé chaud ou froid envoyé à travers une tête de pulvérisation. Cette méthode permet d'évaluer l'angle du spray mais pas sa géométrie ni sa symétrie. Ceci a de plus l'inconvénient d'avoir à prévoir un banc strioscopique, relativement complexe et coûteux, et difficilement adaptable dans une chaine de montage d'un dispositif de pulvérisation de produit fluide, et implique donc soit des tests aléatoires sur une partie seulement des dispositifs fabriqués, soit un ralentissement de la chaine de montage, généralement peu souhaitable.

Les documents EP3047912, JPH0599802 et JPS54127347 décrivent d'autres dispositifs de l'état de la technique.

La présente invention a pour but de surmonter les inconvénients susmentionnés.

En particulier, la présente invention a pour but de fournir un dispositif et un procédé d'analyse d'un dispositif de pulvérisation de produit fluide pharmaceutique qui soit non destructeur des dispositifs testés.

La présente invention a également pour but de fournir un dispositif et un procédé d'analyse qui soit automatisé en grande partie.

La présente invention a aussi pour but de fournir un dispositif et un procédé d'analyse qui permette de tester 100% des dispositifs de pulvérisation, sans ralentir la chaine de montage de manière substantielle.

La présente invention a également pour but de fournir un dispositif et un procédé d'analyse qui soit simple et/ou peu coûteux à fabriquer, à assembler et à utiliser.

La présente a donc pour objet un procédé d'analyse d'un dispositif de pulvérisation de produit fluide pharmaceutique, comprenant les étapes suivantes:
- fournir une tête de pulvérisation d'un dispositif de pulvérisation de produit fluide pharmaceutique, ladite tête de pulvérisation comportant un orifice de pulvérisation,
- fournir une surface de réception comportant une pluralité de zones de contact discrètes séparées par des vides, lesdites zones de contact étant enduites d'un matériau thermosensible,
- amener ladite surface de réception à une température T2,
- faire passer un flux de gaz comprimé à travers ledit orifice de pulvérisation de ladite tête de pulvérisation, ledit flux de gaz comprimé étant à une température T1, différente de T2,
- envoyer ledit flux de gaz comprimé à température T1 sur ladite surface de réception à température T2,
- visualiser la zone d'impact dudit flux de gaz comprimé sur ladite surface de réception, et
- analyser ladite visualisation de ladite zone d'impact pour déterminer si ladite zone d'impact est conforme ou non conforme à des spécifications prédéterminées.

La présente a également pour objet un procédé d'analyse d'un dispositif de pulvérisation de produit fluide pharmaceutique, comprenant les étapes suivantes:
- fournir une tête de pulvérisation d'un dispositif de pulvérisation de produit fluide pharmaceutique, ladite tête de pulvérisation comportant un orifice de pulvérisation,
- fournir une surface de réception comportant une pluralité de zones de contact discrètes séparées par des vides,
- amener ladite surface de réception à une température T2,
- faire passer un flux de gaz comprimé à travers ledit orifice de pulvérisation de ladite tête de pulvérisation, ledit flux de gaz comprimé étant à une température T1, différente de T2,
- envoyer ledit flux de gaz comprimé à température T1 sur ladite surface de réception à température T2,
- visualiser la zone d'impact dudit flux de gaz comprimé sur ladite surface de réception au moyen d'une caméra thermique, et
- analyser ladite visualisation de ladite zone d'impact pour déterminer si ladite zone d'impact est conforme ou non conforme à des spécifications prédéterminées.

Avantageusement, ledit flux de gaz comprimé est un flux d'air comprimé.

Avantageusement, ladite étape d'analyser comprend de déterminer la géométrie, notamment la symétrie, de la zone d'impact dudit flux de gaz comprimé sur ladite surface de réception.

Avantageusement, lesdites spécifications prédéterminées comprennent une étendue planaire prédéterminée de la zone d'impact dudit flux de gaz comprimé sur ladite surface de réception, de sorte que les têtes de pulvérisation pour lesquelles ladite étendue planaire est similaire à ladite étendue planaire prédéterminée sont classées conformes, et les têtes de pulvérisation pour lesquelles ladite étendue planaire est différente de ladite étendue planaire prédéterminée sont classés non conformes.

Avantageusement, un cycle d'utilisation comprend les étapes suivantes:
- envoyer ledit flux de gaz comprimé à température T1 sur ladite surface de réception à température T2,
- prendre une première image de ladite surface de réception au moyen d'une caméra,
- envoyer un flux d'air secondaire à température T2 sur ladite zone de réception,
- prendre une seconde image de ladite surface de réception, et
- soustraire la seconde image de la première image pour visualiser la zone d'impact.

Avantageusement, un cycle d'utilisation comprend les étapes suivantes:
- envoyer un flux d'air secondaire à température T2 sur ladite zone de réception,
- prendre une première image de ladite surface de réception au moyen d'une caméra,
- envoyer ledit flux de gaz comprimé à température T1 sur ladite surface de réception à température T2,
- prendre une seconde image de ladite surface de réception, et
- soustraire la première image de la seconde image pour visualiser la zone d'impact.

La présente a également pour objet un dispositif d'analyse d'un dispositif de pulvérisation de produit fluide pharmaceutique, comprenant:
- une tête de pulvérisation d'un dispositif de pulvérisation de produit fluide pharmaceutique, ladite tête de pulvérisation comportant un orifice de pulvérisation,
- une surface de réception comportant une pluralité de zones de contact discrètes séparées par des vides, lesdites zones de contact étant enduites d'un matériau thermosensible,
- des moyens de régulation de température pour amener ladite surface de réception à une température T2,
- des moyens de génération d'un flux de gaz comprimé pour faire passer un flux de gaz comprimé à travers ledit orifice de pulvérisation de ladite tête de pulvérisation sur ladite surface de réception à température T2, ledit flux de gaz comprimé étant à une température T1, différente de T2,
- des moyens de visualisation, tels qu'une caméra, pour visualiser la zone d'impact dudit flux de gaz comprimé sur ladite surface de réception, et
- des moyens d'analyse pour analyser ladite visualisation de ladite zone d'impact pour déterminer si ladite zone d'impact est conforme ou non conforme à des spécifications prédéterminées.

La présente a également pour objet un dispositif d'analyse d'un dispositif de pulvérisation de produit fluide pharmaceutique, comprenant:
- une tête de pulvérisation d'un dispositif de pulvérisation de produit fluide pharmaceutique, ladite tête de pulvérisation comportant un orifice de pulvérisation,
- une surface de réception comportant une pluralité de zones de contact discrètes séparées par des vides,
- des moyens de régulation de température pour amener ladite surface de réception à une température T2,
- des moyens de génération d'un flux de gaz comprimé pour faire passer un flux de gaz comprimé à travers ledit orifice de pulvérisation de ladite tête de pulvérisation sur ladite surface de réception à température T2, ledit flux de gaz comprimé étant à une température T1, différente de T2,
- une caméra thermique pour visualiser la zone d'impact dudit flux de gaz comprimé sur ladite surface de réception, et
- des moyens d'analyse pour analyser ladite visualisation de ladite zone d'impact pour déterminer si ladite zone d'impact est conforme ou non conforme à des spécifications prédéterminées.

Avantageusement, ledit flux de gaz comprimé est un flux d'air comprimé.

Avantageusement, lesdits moyens de régulation de température comprennent des moyens de génération de flux d'air secondaire pour générer un flux d'air secondaire à ladite température T2.

Avantageusement, lesdits moyens de régulation de température comprennent des fils chauffant et une résistance.

Avantageusement, ladite surface de réception est formée par les extrémités d'une pluralité de pointes disposées en réseau, lesdites extrémités formant lesdites zones de contact.

Avantageusement, lesdites pointes sont solidaires d'une base.

Avantageusement, lesdites pointes sont équidistantes et proches les unes des autres, formant ainsi un réseau régulier et dense de zones de contact sur ladite surface de réception.

Avantageusement, ledit matériau thermosensible est une peinture adaptée à changer de couleur en fonction de la température.

Avantageusement, lesdits moyens de génération du flux de gaz comprimé sont adaptées à générer des impulsions de durée réglable, notamment de 50 à 300 ms.

Ces caractéristiques et avantages et d'autres apparaîtront plus clairement au cours de la description détaillée suivante, faite en référence aux dessins joints, donnés à titre d'exemples non limitatifs, et sur lesquels :
la figure 1 est une vue schématique d'un dispositif pour analyser un dispositif de pulvérisation, selon un mode de réalisation avantageux, en première phase d'un cycle d'utilisation avantageux,
la figure 2 est une vue schématique similaire à celle de la figure 1, en seconde phase dudit cycle d'utilisation,
la figure 3 est un schéma temporel d'un cycle d'utilisation selon un mode de réalisation avantageux,
la figure 4 montre une image thermique obtenue d'une zone d'impact, et
les figures 5 et 6 sont des représentations schématiques de zone d'impact respectivement conforme et non-conforme.

Un objectif de l'invention est d'améliorer la qualité du contrôle des dispositifs de pulvérisation. Pour cela, l'invention prévoit d'analyser 100% des dispositifs, sans ralentissement substantiel de la chaine de montage.

De manière classique, chaque dispositif de pulvérisation comprend une tête de pulvérisation 1 pourvue d'un orifice de pulvérisation 2. Généralement, un profil de pulvérisation (non représenté) est prévu en amont dudit orifice de pulvérisation 2 pour générer un spray en sortie de l'orifice.

La présente invention prévoit de faire passer un flux de gaz comprimé F1 à travers chaque tête de pulvérisation 1, et de diriger ce flux F1, sortant de l'orifice de pulvérisation 2 avec la forme d'un spray conique, vers une surface de réception 10, avantageusement enduite d'un matériau thermosensible capable de changer de couleur au contact du flux F1. Avantageusement, le flux de gaz comprimé F1 est un flux d'air comprimé, mais il est entendu que selon l'invention, n'importe quel gaz approprié autre que de l'air pourrait être utilisé.

Dans le cadre de la présente invention, l'objectif est la mise en évidence d'un flux de gaz comprimé F1 à température T1 différente de la température T2 de la surface de réception 10, son observation sur cette surface de réception 10 au moyen d'une caméra 20, et l'analyse de cette observation par des moyens d'analyse 40.

Le flux de gaz comprimé F1 est à une température T1, et au moment où ce flux F1 est expulsé à travers l'orifice de pulvérisation 2, la surface de réception 10 est à une température T2, différente de T1.

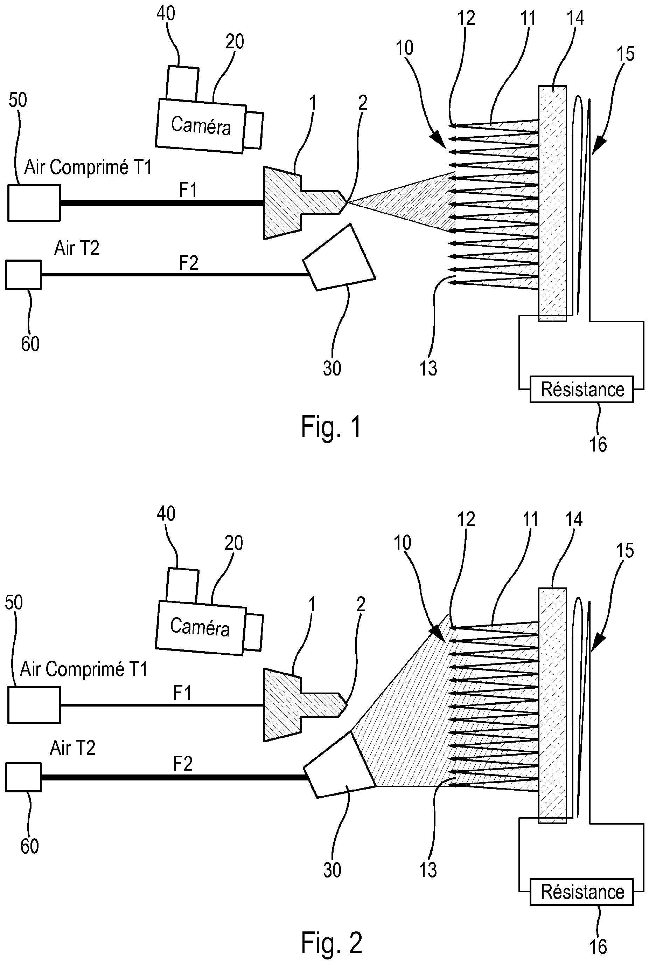

Dans l'exemple des figures 1 à 3, le flux de gaz comprimé F1 est à une température T1 proche de la température ambiante, et la surface de réception 10 est chauffée ou refroidie à une température T2, supérieure ou inférieure à T1. Par exemple, la surface de réception 10 est chauffée à une température T2>T1.

En variante, on pourrait prévoir n'importe quelle combinaison de températures T1 et T2, du moment que la différence entre ces deux températures T1-T2 ou T2-T1 soit suffisamment importante pour être détectée par un matériau thermosensible enduisant la surface de réception 10 et/ou par une caméra thermique. Ainsi par exemple, la surface de réception 10 pourrait être à température ambiante T2 et le flux de gaz comprimé F1 chauffé ou refroidi par rapport à cette température ambiante T2.

La figure 1 montre un dispositif de test selon un mode de réalisation avantageux.

Dans cet exemple, une tête de pulvérisation 1 est disposée en face d'une surface de réception 10. Des moyens de génération 50 d'un flux de gaz comprimé F1 sont prévus pour faire passer un flux de gaz comprimé F1 à température T1 à travers la tête de pulvérisation 1. Une caméra 20 est disposée face à la surface de réception 10, avantageusement à proximité de la tête de pulvérisation 1, pour faire des images de la surface de réception 10.

Des moyens de régulation de température sont prévus pour amener la surface de réception 10 à une température T2 juste avant et/ou juste après l'envoi du flux de gaz comprimé F1. Avantageusement, des moyens de génération 60 de flux d'air secondaire sont prévus pour générer un flux d'air secondaire F2 à température T2, différente de T1. Ce flux d'air secondaire F2 est envoyé sur la surface de réception 10 par une buse 30, en alternance avec le flux de gaz comprimé F1 passant à travers la tête de pulvérisation 1, pour amener ladite surface de réception 10 à ladite température T2 avant et/ou après l'envoi du flux F1. En variante, les moyens de régulation peuvent comporter des fils chauffant 15 et une résistance 16. D'autres moyens de régulation sont possibles. Plusieurs moyens de régulation différents peuvent être associés pour garantir que la surface de réception 10 est à la température T2 au moment où le flux de gaz comprimé F1 à température T1 est envoyé.

La surface de réception 10 forme un plan comporte une pluralité de zones de contact 12 séparées les unes des autres par une pluralité de vides 13. Dans un mode de réalisation avantageux, lesdites zones de contact 12 sont enduites avec un matériau thermosensible.

Dans l'exemple représenté, la surface de réception 10 est formée par les extrémités d'une pluralité de pointes 11 disposées en réseau. Ces extrémités forment alors les zones de contact 12. Ces pointes 11 peuvent être solidaires d'une base 14. Avantageusement, des moyens de régulation de température 15 peuvent agir sur ladite base 14.

Avantageusement, les pointes 11 sont équidistantes, et proches les unes des autres, formant ainsi un réseau régulier et dense de zones de contact 12 sur la surface de réception 10. Plus il y a de pointes 11 et plus les zones de contact 12 sont petites, plus la définition de la zone d'impact du flux de gaz comprimé F1 sur la surface de réception 10 est précise et la forme de cette zone d'impact bien retranscrite.

En variante aux pointes 11, on pourrait utiliser une plaque perforée ou une grille pour former la surface de réception 10, avec dans ce cas les parties séparant les trous formant les zones de contact 12.

Le matériau thermosensible utilisé pour enduire les zones de contact 12 peut être une peinture adaptée à changer de couleur en fonction de la température. Ainsi, lorsque la surface de réception 10 est à la température T2, les zones de contact 12 peuvent avoir une première couleur, et lorsque le flux de gaz comprimé F1 à température T1 arrive sur la surface de réception 10, les zones de contact 12 touchées par ledit flux F1 changent de couleur, permettant de visualiser la zone d'impact du flux F1 sur la surface de réception 10. La caméra 20 permet alors de prendre des images, notamment des images thermiques, de cette zone d'impact.

Dans un autre mode de réalisation, les zones de contact 12 ne sont pas recouvertes avec un matériau thermosensible, et la caméra 20 est une caméra thermique capable de détecter les différences de température entre le flux de gaz comprimé F1 à température T1 et la zone de réception 10 à température T2.

La forme particulière de la surface de réception 10, avec une pluralité de zones de contact discrètes 12 séparées par des vides 13 permet un contact local du flux de gaz comprimé F1 sur les zones de contact 12, sans dispersions et sans perturbations du flux, ce qui rend visible la zone d'impact avec une grande fiabilité.

Pour réaliser les évaluations de conformité, on prévoit avantageusement des moyens d'analyse 40 pour analyser les images de la caméra 20 et ainsi déterminer si la zone d'impact du flux de gaz comprimé F1 issu de ladite tête de pulvérisation 1 sur la surface de réception 10 est conforme ou non conforme à des spécifications prédéterminées. En particulier, on peut prévoir de soustraire l'image de la surface de réception 10 juste avant l'envoi du flux F1 de l'image de la surface de réception 10 juste après l'envoi du flux F1, ce qui permet d'isoler la zone d'impact du flux F1. En variante, on peut prévoir de soustraire l'image après l'envoi du flux d'air secondaire F2 à température T2 de l'image juste après l'envoi du flux de gaz comprimé F1 à température T1.

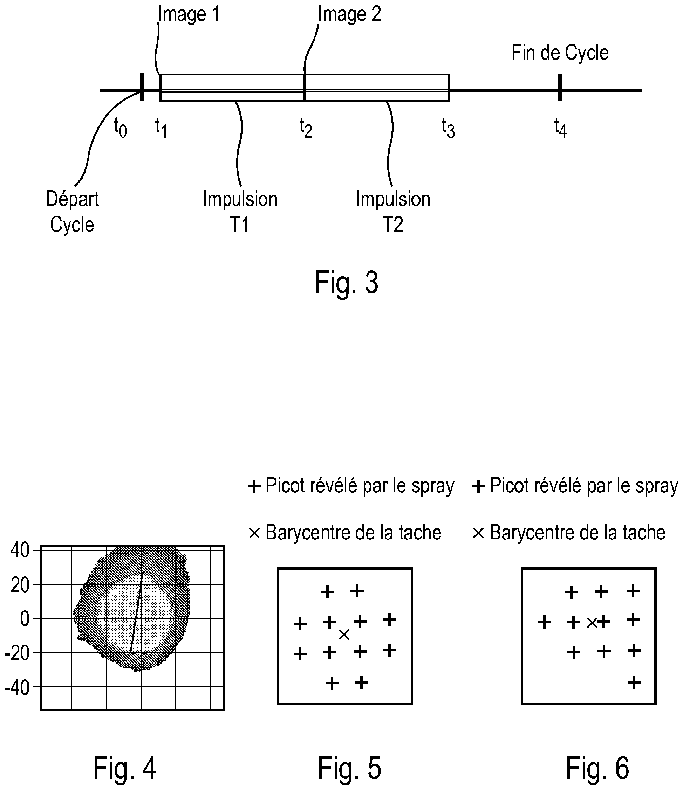

La figure 3 montre un cycle d'utilisation avantageux, avec une première impulsion de flux de gaz comprimé F1 à température T1 envoyée sur la surface de réception 10 au temps t₁, et la prise d'une première image réalisée au même moment t₁. Ensuite, au temps t₂, le flux d'air secondaire F2 à température T2 est envoyé sur la surface de réception 10, avec au même moment la prise d'une seconde image.

En variante, la première image pourrait être prise après envoi du flux d'air secondaire F2 à température T2 et la seconde image au moment de l'envoi du flux de gaz comprimé F1 à température T1.

La durée de l'impulsion de gaz comprimé F1 est avantageusement réglable, notamment de 50 à 300 ms. La durée du flux d'air secondaire F2 est avantageusement aussi réglable.

Avantageusement, on réalise plusieurs cycles successifs sur une même tête de pulvérisation, par exemple cinq cycles. La constance ou répétabilité des résultats permet également d'évaluer la conformité de ladite tête de pulvérisation.

Les spécifications prédéterminées peuvent comprendre une étendue planaire prédéterminée de ladite zone d'impact sur ladite surface de support 10, de sorte que les têtes de pulvérisation 1 pour lesquelles ladite étendue planaire est similaire à ladite étendue planaire prédéterminée sont classées conformes, et les têtes de pulvérisation 1 pour lesquelles ladite étendue planaire est différente de ladite étendue planaire prédéterminée sont classés non conformes. La géométrie, et notamment la symétrie, de la zone d'impact peut aussi être utilisée dans l'évaluation de conformité. D'autres paramètres peuvent aussi être envisagés.

Les moyens d'analyse 40 peuvent comprendre des moyens de mesure de la géométrie de la zone d'impact du flux de gaz comprimé F1 sur la zone de réception 10. Par exemple, on détermine le barycentre de la zone d'impact, et on mesure les distances maximale et minimale de ce barycentre du bord de la zone d'impact. La comparaison de ces distances avec des valeurs prédéterminées permet alors d'évaluer la conformité du dispositif testé. Ainsi, l'évaluation de conformité tient compte non seulement de la surface de la zone d'impact, mais aussi de sa géométrie, en particulier sa symétrie. Ceci permet d'établir qu'un spray sortant d'une tête de pulvérisation conforme aura une forme conique acceptable, tant d'un point de vue de l'angle du spray que de sa symétrie. Avantageusement, on prévoit un ajustement de la température T1 et du débit du flux de gaz comprimé F1 pour adapter au mieux la mesure de la zone d'impact, à une distance donnée de l'orifice de pulvérisation du dispositif testé, par rapport à ce qui pourrait être obtenu avec un spray de liquide. Cela permet de définir un paramétrage permettant de prédire au mieux les performances de chaque tête de pulvérisation dans son utilisation finale, à savoir la distribution de liquide sous forme de spray.

Eventuellement, des moyens de traitement d'image peuvent être utilisés pour réaliser ce type d'analyse.

Les figures 4 et 5 illustrent une image et une représentation schématique obtenues avec le procédé et le dispositif de l'invention, sur lesquelles il est possible d'évaluer l'étendue planaire et la géométrie, notamment la symétrie, de la zone d'impact.

La présente invention présente de nombreux avantages, et notamment:
- elle permet un contrôle automatisé de conformité sur divers types de dispositif de pulvérisation;
- elle permet une analyse non destructive desdits dispositifs de pulvérisation;
- elle permet d'analyser 100% des dispositifs de pulvérisation assemblés sur une chaine de montage, sans ralentissement substantiel de celle-ci;
- elle permet de réaliser plusieurs tests successifs sur un même dispositif pour évaluer la répétabilité des résultats;
- elle utilise un montage compacte et facilement adaptable;
- elle utilise des composants simples et standards, donc généralement peu coûteux;
- elle permet un traitement d'image robuste, qui peut être réalisé en temps réel;
- elle assure une bonne répétabilité et une bonne discrimination des dispositifs conformes et non-conformes.

La présente invention a été décrite en référence à plusieurs modes de réalisation avantageux, mais il est entendu qu'un homme du métier peut y apporter toutes modifications, sans sortir du cadre de la présente invention tel que défini par les revendications annexées.

## Revendications

1. Procédé d'analyse d'un dispositif de pulvérisation de produit fluide pharmaceutique comprenant les étapes suivantes:
- fournir une tête de pulvérisation (1) d'un dispositif de pulvérisation de produit fluide pharmaceutique, ladite tête de pulvérisation (1) comportant un orifice de pulvérisation (2),
- fournir une surface de réception (10),
- amener ladite surface de réception (10) à une température T2,
- faire passer un flux de gaz comprimé (F1) à travers ledit orifice de pulvérisation (2) de ladite tête de pulvérisation (1), ledit flux de gaz comprimé (F1) étant à une température T1, différente de T2,
- envoyer ledit flux de gaz comprimé (F1) à température T1 sur ladite surface de réception (10) à température T2,
- visualiser la zone d'impact dudit flux de gaz comprimé (F1) sur ladite surface de réception (10), et
- analyser ladite visualisation de ladite zone d'impact pour déterminer si ladite zone d'impact est conforme ou non conforme à des spécifications prédéterminées,
le procédé d'analyse étant **caractérisé en ce que** ladite surface de réception (10) comporte une pluralité de zones de contact discrètes séparées par des vides, lesdites zones de contact étant enduites d'un matériau thermosensible (9).

2. Procédé d'analyse d'un dispositif de pulvérisation de produit fluide pharmaceutique, comprenant les étapes suivantes:
- fournir une tête de pulvérisation (1) d'un dispositif de pulvérisation de produit fluide pharmaceutique, ladite tête de pulvérisation (1) comportant un orifice de pulvérisation (2),
- fournir une surface de réception (10),
- amener ladite surface de réception (10) à une température T2,
- faire passer un flux de gaz comprimé (F1) à travers ledit orifice de pulvérisation (2) de ladite tête de pulvérisation (1), ledit flux de gaz comprimé (F1) étant à une température T1, différente de T2,
- envoyer ledit flux de gaz comprimé (F1) à température T1 sur ladite surface de réception (10) à température T2,
- visualiser la zone d'impact dudit flux de gaz comprimé (F1) sur ladite surface de réception (10) au moyen d'une caméra thermique (20), et
- analyser ladite visualisation de ladite zone d'impact pour déterminer si ladite zone d'impact est conforme ou non conforme à des spécifications prédéterminées,
le procédé d'analyse étant **caractérisé en ce que** ladite surface de réception (10) comporte une pluralité de zones de contact discrètes séparées par des vides,

3. Procédé selon la revendication 1 ou 2, dans lequel ledit flux de gaz comprimé (F1) est un flux d'air comprimé.

4. Procédé selon l'une quelconque des revendications précédentes, dans lequel ladite étape d'analyser comprend de déterminer la géométrie, notamment la symétrie, de la zone d'impact dudit flux de gaz comprimé (F1) sur ladite surface de réception (10).

5. Procédé selon l'une quelconque des revendications précédentes, dans lequel lesdites spécifications prédéterminées comprennent une étendue planaire prédéterminée de la zone d'impact dudit flux de gaz comprimé (F1) sur ladite surface de réception (10), de sorte que les têtes de pulvérisation (1) pour lesquelles ladite étendue planaire est similaire à ladite étendue planaire prédéterminée sont classées conformes, et les têtes de pulvérisation (1) pour lesquelles ladite étendue planaire est différente de ladite étendue planaire prédéterminée sont classées non conformes.

6. Procédé selon l'une quelconque des revendications précédentes, dans lequel un cycle d'utilisation comprend les étapes suivantes:
- envoyer ledit flux de gaz comprimé (F1) à température T1 sur ladite surface de réception (10) à température T2,
- prendre une première image de ladite surface de réception (10) au moyen d'une caméra (20),
- envoyer un flux d'air secondaire (F2) à température T2 sur ladite zone de réception (10),
- prendre une seconde image de ladite surface de réception (10), et
- soustraire la seconde image de la première image pour visualiser la zone d'impact.

7. Procédé selon l'une quelconque des revendications précédentes, dans lequel un cycle d'utilisation comprend les étapes suivantes:
- envoyer un flux d'air secondaire (F2) à température T2 sur ladite zone de réception (10),
- prendre une première image de ladite surface de réception (10) au moyen d'une caméra (20),
- envoyer ledit flux de gaz comprimé (F1) à température T1 sur ladite surface de réception (10) à température T2,
- prendre une seconde image de ladite surface de réception (10), et
- soustraire la première image de la seconde image pour visualiser la zone d'impact.

8. Dispositif d'analyse d'un dispositif de pulvérisation de produit fluide pharmaceutique comprenant :
- une tête de pulvérisation (1) d'un dispositif de pulvérisation de produit fluide pharmaceutique, ladite tête de pulvérisation (1) comportant un orifice de pulvérisation (2),
- une surface de réception (10),
- des moyens de régulation de température pour amener ladite surface de réception (10) à une température T2,
- des moyens de génération (50) d'un flux de gaz comprimé (F1) pour faire passer un flux de gaz comprimé (F1) à travers ledit orifice de pulvérisation (2) de ladite tête de pulvérisation (1) sur ladite surface de réception (10) à température T2, ledit flux de gaz comprimé (F1) étant à une température T1, différente de T2,
- des moyens de visualisation (20), tels qu'une caméra, pour visualiser la zone d'impact dudit flux de gaz comprimé (F1) sur ladite surface de réception (10), et
- des moyens d'analyse (40) pour analyser ladite visualisation de ladite zone d'impact pour déterminer si ladite zone d'impact est conforme ou non conforme à des spécifications prédéterminées,
le dispositif d'analyse étant **caractérisé en ce que** ladite surface de réception (10) comporte une pluralité de zones de contact discrètes séparées par des vides,
lesdites zones de contact étant enduites d'un matériau thermosensible (9).

9. Dispositif d'analyse d'un dispositif de pulvérisation de produit fluide pharmaceutique comprenant :
- une tête de pulvérisation (1) d'un dispositif de pulvérisation de produit fluide pharmaceutique, ladite tête de pulvérisation (1) comportant un orifice de pulvérisation (2),
- une surface de réception (10),
- des moyens de régulation de température pour amener ladite surface de réception (10) à une température T2,
- des moyens de génération (50) d'un flux de gaz comprimé (F1) pour faire passer un flux de gaz comprimé (F1) à travers ledit orifice de pulvérisation (2) de ladite tête de pulvérisation (1) sur ladite surface de réception (10) à température T2, ledit flux de gaz comprimé (F1) étant à une température T1, différente de T2,
- une caméra thermique (20) pour visualiser la zone d'impact dudit flux de gaz comprimé (F1) sur ladite surface de réception (10), et
- des moyens d'analyse (40) pour analyser ladite visualisation de ladite zone d'impact pour déterminer si ladite zone d'impact est conforme ou non conforme à des spécifications prédéterminées,
le dispositif d'analyse étant **caractérisé en ce que** la surface de réception (10) comporte une pluralité de zones de contact discrètes séparées par des vides.

10. Dispositif selon la revendication 8 ou 9, dans lequel ledit flux de gaz comprimé (F1) est un flux d'air comprimé.

11. Dispositif selon l'une quelconque des revendications 8 à 10, dans lequel lesdits moyens de régulation de température comprennent des moyens de génération (60) de flux d'air secondaire pour générer un flux d'air secondaire (F2) à ladite température T2.

12. Dispositif selon l'une quelconque des revendications 8 à 11, dans lequel lesdits moyens de régulation de température comprennent des fils chauffant (15) et une résistance (16).

13. Dispositif selon l'une quelconque des revendications 8 à 12, dans lequel ladite surface de réception (10) est formée par les extrémités d'une pluralité de pointes (11) disposées en réseau, lesdites extrémités formant lesdites zones de contact (12).

14. Dispositif selon la revendication 13, dans lequel lesdites pointes (11) sont solidaires d'une base (14).

15. Dispositif selon la revendication 13 ou 14, dans lequel lesdites pointes (11) sont équidistantes et proches les unes des autres, formant ainsi un réseau régulier et dense de zones de contact (12) sur ladite surface de réception (10).

16. Dispositif selon la revendication 8 ou selon l'une quelconque des revendications 10 à 15 lorsque dépendante de la revendication 8, dans lequel ledit matériau thermosensible est une peinture adaptée à changer de couleur en fonction de la température.

17. Dispositif selon l'une quelconque des revendications 8 à 16, dans lequel lesdits moyens de génération (50) du flux de gaz comprimé (F1) sont adaptées à générer des impulsions de durée réglable, notamment de 50 à 300 ms.

## Patentansprüche

1. Verfahren zur Analyse einer Sprühvorrichtung für ein flüssiges pharmazeutisches Produkt, folgende Schritte umfassend:
- Bereitstellen eines Sprühkopfs (1) einer Sprühvorrichtung für ein flüssiges pharmazeutisches Produkt, wobei der Sprühkopf (1) eine Sprühöffnung (2) aufweist,
- Bereitstellen einer Aufnahmefläche (10),
- Einstellen der Aufnahmefläche (10) auf eine Temperatur T2,
- Leiten eines Druckgasstroms (F1) durch die Sprühöffnung (2) des Sprühkopfes (1), wobei die Temperatur T1 des Druckgasstroms (F1) ungleich der Temperatur T2 ist,
- Zuführen des Druckgasstroms (F1) mit der Temperatur T1 zur Aufnahmefläche (10) mit der Temperatur T2,
- Visualisieren des Auftreffbereichs des Druckgasflusses (F1) auf der Aufnahmefläche (10), und
- Analysieren der Visualisierung des Auftreffbereichs zur Bestimmung, ob der Auftreffbereich mit vorbestimmten Spezifikationen konform ist oder nicht,
wobei das Analyseverfahren **dadurch gekennzeichnet ist, dass** die Aufnahmefläche (10) eine Vielzahl von durch Lücken voneinander getrennten diskreten Kontaktbereichen umfasst, wobei diese Kontaktbereiche mit einem wärmeempfindlichen Material (9) beschichtet sind.

2. Verfahren zur Analyse einer Sprühvorrichtung für ein flüssiges pharmazeutisches Produkt, folgende Schritte umfassend:
- Bereitstellen eines Sprühkopfs (1) einer Sprühvorrichtung für ein flüssiges pharmazeutisches Produkt, wobei der Sprühkopf (1) eine Sprühöffnung (2) aufweist,
- Bereitstellen einer Aufnahmefläche (10),
- Einstellen der Aufnahmefläche (10) auf eine Temperatur T2,
- Leiten eines Druckgasstroms (F1) durch die Sprühöffnung (2) des Sprühkopfes (1), wobei die Temperatur (T1) des Druckgasstroms (F1) ungleich der Temperatur T2 ist,
- Zuführen des Druckgasstroms (F1) mit der Temperatur T1 zur Aufnahmefläche (10) mit der Temperatur T2,
- Visualisieren des Auftreffbereichs des Druckgasstroms (F1) auf der Aufnahmefläche (10) mittels einer Wärmebildkamera (20), und
- Analysieren der Visualisierung des Auftreffbereichs zur Bestimmung, ob der Auftreffbereich mit vorbestimmten Spezifikationen konform ist oder nicht,
wobei das Analyseverfahren **dadurch gekennzeichnet ist, dass** die Aufnahmefläche (10) eine Vielzahl von durch Lücken voneinander getrennten diskreten Kontaktzonen umfasst.

3. Verfahren nach Anspruch 1 oder 2, bei dem es sich bei dem Druckgasfluss (F1) um einen Druckluftstrom handelt.

4. Verfahren nach einem der vorhergehenden Ansprüche, bei dem der Analyseschritt das Bestimmen der Geometrie, insbesondere der Symmetrie, des Auftreffbereichs des Druckgasstroms (F1) auf der Aufnahmefläche (10) umfasst.

5. Verfahren nach einem der vorhergehenden Ansprüche, bei dem die vorbestimmten Spezifikationen eine vorbestimmte flächige Ausdehnung des Auftreffbereichs des Druckgasstroms (F1) auf der Aufnahmefläche (10) umfassen, sodass diejenigen Sprühköpfe (1), bei denen die flächige Ausdehnung ähnlich der vorbestimmten flächigen Ausdehnung ist, als konform eingestuft werden, und diejenigen Sprühköpfe (1), bei denen die flächige Ausdehnung von der vorbestimmten flächigen Ausdehnung abweicht, als nicht-konform eingestuft werden.

6. Verfahren nach einem der vorhergehenden Ansprüche, bei dem ein Anwendungszyklus folgende Schritte umfasst:
- Leiten des Druckgasstroms (F1) mit der Temperatur T1 auf die Aufnahmefläche (10) mit der Temperatur T2,
- Aufnehmen eines ersten Bildes besagter Aufnahmefläche (10) mittels einer Kamera (20),
- Zuführen eines sekundären Luftstroms (F2) mit einer Temperatur von T2 zum Aufnahmebereich (10),
- Aufnehmen eines zweiten Bilds der Aufnahmefläche (10), und
- Subtrahieren des zweiten Bilds von dem ersten Bild zur Visualisierung des Auftreffbereichs.

7. Verfahren nach einem der vorhergehenden Ansprüche, bei dem ein Anwendungszyklus folgende Schritte umfasst:
- Zuführen eines sekundären Luftstroms (F2) mit einer Temperatur von T2 zum Aufnahmebereich (10),
- Aufnehmen eines ersten Bilds der Aufnahmefläche (10) mittels einer Kamera (20),
- Zuführen des Druckgasstroms (F1) mit der Temperatur T1 zur Aufnahmefläche (10) mit der Temperatur T2,
- Aufnehmen eines zweiten Bilds der Aufnahmefläche (10), und
- Subtrahieren des ersten Bilds von dem zweiten Bild zur Visualisierung des Auftreffbereichs.

8. Vorrichtung zur Analyse einer Sprühvorrichtung für ein flüssiges pharmazeutisches Produkt, umfassend:
- einen Sprühkopf (1) einer Sprühvorrichtung für ein flüssiges pharmazeutisches Produkt, wobei der Sprühkopf (1) eine Sprühöffnung (2) aufweist,
- eine Aufnahmefläche (10),
- Mittel zur Temperaturregelung für das Einstellen der Aufnahmefläche (10) auf eine Temperatur T2,
- Mittel (50) zur Erzeugung eines Druckgasstroms (F1), mit denen ein Druckgasstrom (F1) durch die Sprühöffnung (2) des Sprühkopfes (1) hindurch der Aufnahmefläche (10) mit einer Temperatur T2 zugeführt wird, wobei die Temperatur (T1) des Druckgasstroms (F1) ungleich der Temperatur T2 ist,
- Visualisierungsmittel (20), beispielsweise eine Kamera, zum Visualisieren des Auftreffbereichs des Druckgasstroms (F1) auf der Aufnahmefläche (10), und
- Analysemittel (40) zum Analysieren der Visualisierung des Auftreffbereichs zur Bestimmung, ob der Auftreffbereich mit vorbestimmten Spezifikationen konform ist oder nicht,
wobei die Analysevorrichtung **dadurch gekennzeichnet ist, dass** die Aufnahmefläche (10) eine Vielzahl von durch Lücken voneinander getrennten diskreten Kontaktbereichen aufweist, wobei die Kontaktbereiche mit einem wärmeempfindlichen Material (9) beschichtet sind.

9. Vorrichtung zur Analyse einer Sprühvorrichtung für ein flüssiges pharmazeutisches Produkt, umfassend:
- einen Sprühkopf (1) einer Sprühvorrichtung für ein flüssiges pharmazeutisches Produkt, wobei der Sprühkopf (1) eine Sprühöffnung (2) aufweist,
- eine Aufnahmefläche (10),
- Mittel zur Temperaturregelung zum Einstellen der Aufnahmefläche (10) auf eine Temperatur T2,
- Mittel (50) zur Erzeugung eines Druckgasstroms (F1), mit denen ein Druckgasstrom (F1) durch die Sprühöffnung (2) des Sprühkopfes (1) hindurch der Aufnahmefläche (10) mit einer Temperatur T2 zugeführt wird, wobei die Temperatur (T1) des Druckgasstroms (F1) ungleich der Temperatur T2 ist,
- eine Wärmebildkamera (20) zur Visualisierung des Auftreffbereichs des Druckgasstroms (F1) auf der Aufnahmefläche (10), und
- Analysemittel (40) zum Analysieren der Visualisierung des Auftreffbereichs zur Bestimmung, ob der Auftreffbereich mit vorbestimmten Spezifikationen konform ist oder nicht, wobei die Analysevorrichtung **dadurch gekennzeichnet ist, dass** die Aufnahmefläche (10) eine Vielzahl von durch Lücken voneinander getrennten diskreten Kontaktbereichen aufweist.

10. Vorrichtung nach Anspruch 8 oder 9, bei der es sich bei dem Druckgasstrom (F1) um einen Druckluftstrom handelt.

11. Vorrichtung nach einem der Ansprüche 8 bis 10, bei der die Mittel zur Temperaturregelung Mittel (60) zur Erzeugung eines Sekundärluftstroms umfassen, mit denen ein Sekundärluftstrom (F2) mit der Temperatur T2 erzeugt wird.

12. Vorrichtung nach einem der Ansprüche 8 bis 11, bei der die Mittel zur Temperaturregelung Heizdrähte (15) und einen Widerstand (16) umfassen.

13. Vorrichtung nach einem der Ansprüche 8 bis 12, bei der die Aufnahmefläche (10) durch die Enden einer Vielzahl von netzartig angeordneten Spitzen (11) gebildet wird, wobei diese Enden die Kontaktbereiche (12) bilden.

14. Vorrichtung nach Anspruch 13, bei der die Spitzen (11) fest mit einer Basis (14) verbunden sind.

15. Vorrichtung nach Anspruch 13 oder 14, bei der die Spitzen (11) unter gleichem Abstand und nahe beieinander angeordnet sind, wodurch sie ein regelmäßiges und dichtes Netz von Kontaktbereichen (12) auf der Aufnahmefläche (10) bilden.

16. Vorrichtung nach Anspruch 8 oder nach einem der Ansprüche 10 bis 15 sofern abhängig von Anspruch 8, bei der es sich bei dem wärmeempfindlichen Material um einen Lack handelt, der zur Farbänderung in Abhängigkeit von der Temperatur ausgelegt ist.

17. Vorrichtung nach einem der Ansprüche 8 bis 16, bei der die Mittel (50) zur Erzeugung des Druckgasstroms (F1) zur Erzeugung von Impulsen von einstellbarer Dauer, insbesondere von 50 bis 300 ms, ausgelegt sind.

## Claims

1. A method for analysing a device for spraying a pharmaceutical fluid product comprising the following steps:
- providing a spray head (1) for a device for spraying a pharmaceutical fluid product, said spray head (1) comprising a spray orifice (2),
- providing a receiving surface (10),
- bringing said receiving surface (10) to a temperature T2,
- passing a flow of compressed gas (F1) through said spray orifice (2) of said spray head (1), said flow of compressed gas (F1) being at a temperature T1 which differs from T2,
- sending said flow of compressed gas (F1) at temperature T1 onto said receiving surface (10) at temperature T2,
- visualising the impact zone for said flow of compressed gas (F1) on said receiving surface (10), and
- analysing said visualisation of said impact zone in order to determine whether or not said impact zone complies with predetermined specifications, the method for analysing being **characterized in that** said receiving surface (10) comprises a plurality of discrete contact zones separated by voids, said contact zones being coated with a heat sensitive material (9).

2. A method for analysing a device for spraying a pharmaceutical fluid product, comprising the following steps:
- providing a spray head (1) for a device for spraying a pharmaceutical fluid product, said spray head (1) comprising a spray orifice (2),
- providing a receiving surface (10),
- bringing said receiving surface (10) to a temperature T2,
passing a flow of compressed gas (F1) through said spray orifice (2) of said spray head (1), said flow of compressed gas (F1) being at a temperature T1 which differs from T2,
- sending said flow of compressed gas (F1) at temperature T1 onto said receiving surface (10) at temperature T2,
- visualising the impact zone for said flow of compressed gas (F1) on said receiving surface (10) by means of a thermal camera (20), and
- analysing said visualisation of said impact zone in order to determine whether or not said impact zone complies with predetermined specifications, the method for analysing being **characterized in that** said receiving surface (10) comprises a plurality of discrete contact zones separated by voids.

3. A method as claimed in claim 1 or claim 2, in which said flow of compressed gas (F1) is a flow of compressed air.

4. The method as claimed in any one of the preceding claims, in which said step for analysis comprises determining the geometry, in particular the symmetry, of the impact zone for said flow of compressed gas (F1) on said receiving surface (10).

5. The method as claimed in any one of the preceding claims, in which said predetermined specifications comprise a predetermined planar extent of the impact zone for said flow of compressed gas (F1) on said receiving surface (10), in a manner such that the spray heads (1) for which said planar extent is similar to said predetermined planar extent are classified as compliant, and the spray heads (1) for which said planar extent is different from said predetermined planar extent are classified as non-compliant.

6. The method as claimed in any one of the preceding claims, in which an operating cycle comprises the following steps:
- sending said flow of compressed gas (F1) at temperature T1 onto said receiving surface (10) at temperature T2,
- taking a first image of said receiving surface (10) by means of a camera (20),
- sending a secondary flow of air (F2) at temperature T2 onto said receiving zone (10),
- taking a second image of said receiving surface (10), and
- subtracting the second image from the first image in order to visualise the impact zone.

7. The method as claimed in any one of the preceding claims, in which an operating cycle comprises the following steps:
- sending a secondary flow of air (F2) at temperature T2 onto said receiving zone (10),
- taking a first image of said receiving surface (10) by means of a camera (20),
- sending said flow of compressed gas (F1) at temperature T1 onto said receiving surface (10) at temperature T2,
- taking a second image of said receiving surface (10), and
- subtracting the first image from the second image in order to visualise the impact zone.

8. A device for analysing a device for spraying a pharmaceutical fluid product comprising:
- a spray head (1) for a device for spraying a pharmaceutical fluid product, said spray head (1) comprising a spray orifice (2);
- a receiving surface (10),
- temperature regulating means for bringing said reception surface (10) to a temperature T2,
- means (50) for generating a flow of compressed gas (F1) in order to pass a flow of compressed gas (F1) through said spray orifice (2) of said spray head (1) onto said receiving surface (10) at temperature T2, said flow of compressed gas (F1) being at a temperature T1 which differs from T2,
- visualisation means (20), such as a camera, for visualising the impact zone for said flow of compressed gas (F1) on said receiving surface (10), and
- means (40) for analysis for the analysis of said visualisation of said impact zone in order to determine whether or not said impact zone complies with predetermined specifications, the device for analysing being **characterized in that** said receiving surface (10) comprises a plurality of discrete contact zones separated by voids, said contact zones being coated with a heat sensitive material (9).

9. A device for analysing a device for spraying a pharmaceutical fluid product comprising:
- a spray head (1) for a device for spraying a pharmaceutical fluid product, said spray head (1) comprising a spray orifice (2);
- a receiving surface (10),
- temperature regulating means for bringing said reception surface (10) to a temperature T2,
- means (50) for generating a flow of compressed gas (F1) in order to pass a flow of compressed gas (F1) through said spray orifice (2) of said spray head (1) onto said receiving surface (10) at temperature T2, said flow of compressed gas (F1) being at a temperature T1 which differs from T2,
- a thermal camera (20) for visualising the impact zone for said flow of compressed gas (F1) on said receiving surface (10), and
- means (40) for analysis for the analysis of said visualisation of said impact zone in order to determine whether or not said impact zone complies with predetermined specifications, the device for analysing being **characterized in that** said receiving surface (10) comprises a plurality of discrete contact zones separated by voids.

10. The device as claimed in claim 8 or claim 9, in which said flow of compressed gas (F1) is a flow of compressed air.

11. The device as claimed in any one of claims 8 to 10, in which said temperature regulation means comprise means (60) for generating a secondary flow of air in order to generate a secondary flow of air (F2) at said temperature T2.

12. The device as claimed in any one of claims 8 to 11, in which said temperature regulating means comprise heating wires and a resistor (16).

13. The device as claimed in any one of claims 8 to 12, in which said receiving surface (10) is formed by the ends of a plurality of points (11) disposed in an array, said ends forming said contact zones (12).

14. The device as claimed in claim 13, in which said points (11) are integral with a base (14).

15. The device as claimed in claim 13 or claim 14, in which said points (11) are equidistant from and close to one another, thereby forming a regular and dense array of contact zones (12) on said receiving surface (10).

16. The device as claimed in claim 8 or in any one of claims 10 to 15 when dependent on claim 8, in which said heat sensitive material is a paint adapted to change colour as a function of temperature.

17. The device as claimed in any one of claims 8 to 16, in which said means (50) for generating a flow of compressed gas (F1) are adapted to generate pulses of adjustable duration, in particular from 50 to 300 ms.
